(19) Europäisches Patentamt
European Patent Office
Office européen des brevets

(11) Publication number: **0 254 852 B1**

(12) # EUROPEAN PATENT SPECIFICATION

(45) Date of publication of patent specification: **18.09.91**

(51) Int. Cl.5: **C07C 259/04**, C07C 259/08, A61K 31/16

(21) Application number: **87108634.4**

(22) Date of filing: **16.06.87**

(54) **Hydroxamic acid derivatives and pharmaceutical compositions comprising them.**

(30) Priority: **07.07.86 US 883227**

(43) Date of publication of application:
**03.02.88 Bulletin 88/05**

(45) Publication of the grant of the patent:
**18.09.91 Bulletin 91/38**

(84) Designated Contracting States:
**BE CH DE FR GB IT LI NL SE**

(56) References cited:
**FR-A- 1 489 121**
**GB-A- 1 243 038**

**JOURNAL OF THE AMERICAN CHEMICAL SOCIETY, vol. 106, no. 5, 7th March 1984, pages 1503-1504, Columbus, Ohio, US; E.J. COREY et al.: "Rationally designed, potent competitive inhibitors of leukotriene biosynthesis"**

(73) Proprietor: **E.R. Squibb & Sons, Inc.**
**Lawrenceville-Princeton Road**
**Princeton, N.J. 08540-4000(US)**

(72) Inventor: **Haslanger, Martin Frederick**
**53 West Glen Avenue**
**Ridgewood New Jersey(US)**
Inventor: **Gordon, Eric Michael**
**126 Laning Avenue**
**Penington New Jersey(US)**

(74) Representative: **Staub, Gabriella, Dr.-Ing. et al**
**Baaderstrasse 3**
**W-8000 München 5(DE)**

**EP 0 254 852 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to hydroxamic acid derivatives and more particularly concerns such derivatives which are inhibitors of $\Delta^5$-lipoxygenase and as such are useful, for example, as antiallergy agents.

In accordance with the present invention, new hydroxamic acid derivatives useful as $\Delta^5$-lipoxygenase inhibitors are provided. These new compounds have the general formula

I

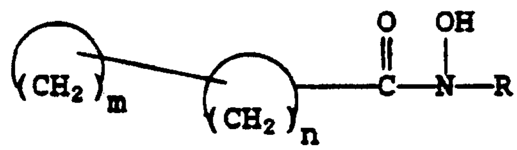

wherein

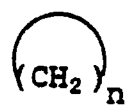 and 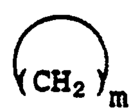

are cycloalkyl groups which may be the same or different; R is hydrogen, lower alkyl, aryl, lower alkenyl, cycloalkyl, or aralkyl; n is an integer from 3 to 12; and m is an integer from 3 to 12. Further in accordance with the present invention, a method for using the above compounds is provided.

The hydroxamic acid derivatives of the present invention may form salts with alkali metals, such as lithium, sodium or potassium. In addition, the compounds of formula I will form salts with dicyclohexylamine or other amines as well as with tris(hydroxymethyl)aminomethane, glucamine and other amines as set out in United States patent 4,294,759.

The term "lower alkyl" or "alkyl" as employed herein by itself or as part of another group includes both straight and branched chain radicals of up to 12 carbons, preferably 1 to 8 carbons, such as methyl, ethyl, propyl, isopropyl, butyl, t-butyl, isobutyl, pentyl, hexyl, isohexyl, heptyl, 4,4-dimethylpentyl, octyl, 2,2,4-trimethylpentyl, nonyl, decyl, undecyl, dodecyl, the various branched chain isomers thereof, and the like as well as such groups including a halo-substituent, such as F, Br, Cl or I or $CF_3$, an alkoxy substituent, an aryl substituent, an alkyl-aryl substituent, a haloaryl substituent, a cycloalkyl substituent, an alkylcycloalkyl substituent, hydroxy, an alkylamino substituent, an alkanoylamino substituent, an arylcarbonylamino substituent, a nitro substituent, a cyano substituent, a thiol substituent or an alkylthio substituent.

The term "cycloalkyl" employed herein by itself or as part of another group includes saturated cyclic hydrocarbon groups containing 3 to 12 carbons, preferably 3 to 8 carbons, which include cyclopropyl, cyclobutyl, cyclopentyl, cyclohexyl, cycloheptyl, cyclooctyl, cyclodecyl and cyclododecyl, which groups are substituted with the same, or a different cycloalkyl, preferably at the 2, 3 or 4 position.

The term "aryl" or "Ar" as employed herein by itself or as part of another group refers to monocyclic or bicyclic aromatic groups containing from 6 to 10 carbons in the ring portion, such as phenyl, naphthyl, substituted phenyl or substituted naphthyl wherein the substituent on either the phenyl or naphthyl may be 1 or 2 lower alkyl groups, 1 or 2 halogens (Cl, Br or F), 1 or 2 lower alkoxy groups, 1 or 2 hydroxyl groups, 1 or 2 alkylamino groups, 1 or 2 alkanoylamino groups, 1 or 2 arylcarbonylamino groups, 1 or 2 amino groups, 1 or 2 nitro groups, 1 or 2 cyano groups, 1 or 2 thiol groups and/or 1 or 2 alkylthio groups.

The term "aralkyl", "aryl-alkyl" or "aryl-lower alkyl" as used herein refers to lower alkyl groups as discussed above having an aryl substituent, such as benzyl.

The term "lower alkenyl" or "alkenyl" as employed herein by itself or as part of another group includes an unsaturated hydrocarbon group having from 3 to 8 carbons and a single carbon-carbon double bond, such as ethenyl, 1-propenyl, 2-propenyl, 1-butenyl, 2-butenyl, 3-butenyl and the like.

The term "lower alkoxy" as employed herein includes the above-defined lower alkyl group linked to an oxygen atom.

The term "acyl" as used herein by itself or as part of another group refers to an alkyl carbonyl or alkenyl carbonyl group.

The term "aroyl" as used herein by itself or as part of another group refers to an aryl carbonyl group.

The term "halogen" or "halo" as used herein refers to chlorine, bromine, fluorine or iodine with chlorine being preferred.

Preferred are those compounds of the invention wherein

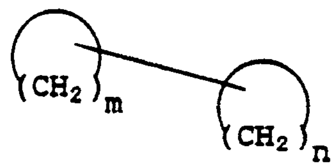

is 4-cyclohexylcyclohexyl and R is lower alkyl, such as methyl or ethyl.

The various compounds of the invention may be prepared as described below.

A carboxylic acid of the formula

II

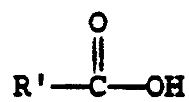

(wherein R′ is an aromatic hydrocarbon substituted with the same, or a different, aromatic hydrocarbon) is put into a solution with an appropriate organic solvent, e.g., ethanol, an acid such as acetic acid or propionic acid, with or without the presence of a trace of a mineral acid, e.g., hydrochloric or sulfuric, and platinum oxide. This solution is first hydrogenated at, or above, atmospheric pressure and at a temperature between about 20° and 100° C, to afford a compound of the formula

III

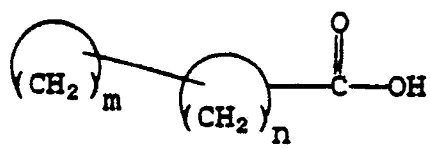

Thereafter, the acid of formula III in a solvent, e.g., tetrahydrofuran, is subjected to a chlorinating agent, e.g., oxalyl chloride or thionyl chloride, to give the acid chloride of the formula

IV

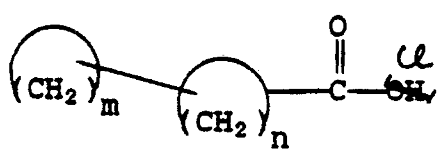

Compound IV is then reacted with one or more parts of an amine of the formula

V

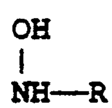

to provide the compound of formula I.

The compounds of the invention are Δ 5-lipoxygenase inhibitors and prevent leukotriene $C_4$ formation in macrophages (Samuelsson, B., Science, Vol. 220, p. 568-575, 1983). The administration of compounds of

this invention to humans or animals provides a method for treating allergy of a reagin or non-reagin nature. Asthma is preferably treated but any allergy wherein leukotrienes are thought to be involved as pharmacological mediators of anaphylaxis can be treated. For example, the compounds of this invention can be used for treatement of such conditions as allergic rhinitis, food allergy and urticaria as well as asthma.

An effective but essentially non-toxic quantity of the compound is employed in treatment.

The compounds of the invention can be administered orally or parenterally to various mammalian species known to be subject to such maladies, e.g., humans, cats, dogs, and the like in an effective amount within the dosage range of about 1 to 100 mg/kg, preferably about 1 to 50 mg/kg and especially about 2 to 25 mg/kg on a regimen in single or 2 to 4 divided daily doses.

The active substance can be utilized in a composition such as tablet, capsule, solution or suspension containing about 5 to about 500 mg per unit of dosage of a compound or mixture of compounds of formula I. They may be compounded in conventional matter with a physiologically acceptable vehicle or carrier, excipient, binder, preservative, stabilizer, flavor, etc. as called for by accepted pharmaceutical practice. Also as indicated in the discussion above, certain members additionally serve as intermediates for other members of the group.

The following examples represent specific embodiment of the present invention.

Example 1
4-Cyclohexyl-N-hydroxy-N-methylcyclohexanecarboxamide

A. 4-Cyclohexyl-cyclohexane-carboxylic acid

A solution of biphenyl-4-carboxylic acid (9.9 g, 50 mmol) in 500 ml of ethanol and 150 ml of glacial acetic acid containing 1.0 g of platinum oxide was hydrogenated overnight at 2.76 bar (40 psi). Upon filtering the solution of concentrating the filtrate under reduced pressure, 10.5 g of bicyclohexyl-4-carboxylic acid was obtained as a white solid (m.p. 122-125° C).

B. 4-Cyclohexyl-N-hydroxy-N-methylcyclohexane-carboxamide

To a solution of 4-cyclohexyl-cyclohexane-carboxylic acid (1.47 g, 7 mmol) in 50 ml of tetrahydrafuran was added oxalyl chloride (90.67 ml, 7.7 mmol) and a few drops of dimethylformamide. After stirring for one hour, the reaction mixture was concentrated to about 1/3 of the original volume and added dropwise into a cold (~0° C), stirred solution of N-methylhydroxylamine hydrochloride (1.17 g, 14 mmol) in 40 ml of 1 N sodium hydroxide. The mixture was stirred for two hours and extracted three times with ethyl acetate. The combined ethyl acetate extracts were washed twice with water, dried with anhydrous sodium sulfate and concentrated. Purification of flash chromatography was crystallization afforded 0.342 g of 4-cyclohexyl-N-hydroxy-N-methylcyclohexane-carboxamide as a white solid (m.p. 89-90° C).

Example 2
4-Cyclohexyl-N-(1,1-dimethylethyl)-N-hydroxy-cyclohexanecarboxamide

A. 4-Cyclohexyl-cyclohexane-carboxylic acid

The 4-cyclohexyl-cyclohexane-carboxylic acid was prepared as in part A of Example 1.

B. 4-Cyclohexyl-N-(1,1-dimethylethyl)-N-hydroxy-cyclohexanecarboxamide

To a solution of 4-cyclohexyl-cyclohexane-carboxylic acid (1.47 g, 7 mmol) in 20 ml of tetrahydrofuran was added oxalyl chloride (0.67 ml, 7.7 mmol) and a few drops of dimethylformamide. After stirring for one hour, the reaction mixture was added dropwise into a cold (~0° C), stirred solution of N-(1,1-dimethylethyl)-hydroxylamine hydrochloride (1.75 g, 14 mmol) in 40 ml of 1 N sodium hydroxide. The mixture was stirred for two hours, from 0° C to room temperature and extracted three times with ethyl acetate. The combined ethyl acetate extracts were then washed twice with water, dried with anhydrous sodium sulfate and concentrated. Purification by flash chromatography provided 0.186 g of the 4-cyclohexyl-N-(1,1-dimethylethyl)-N-hydroxy-cyclohexane-carboxamide as a white solid (m.p. 119-120° C).

Examples 3 to 20

The following additional compounds within the scope of the present invention may be prepared employing the teachings as outlined above and in the working Examples.

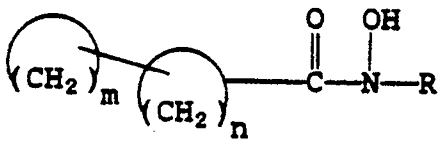

| Example No. | 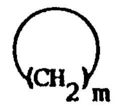 | 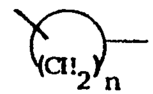 | R |
|---|---|---|---|
| 3 | 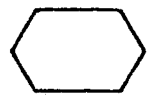 | 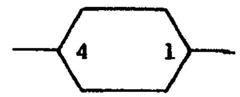 | H |
| 4 | 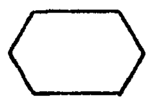 | 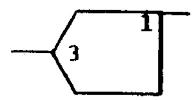 | $C_2H_5$ |
| 5 | 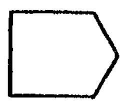 | 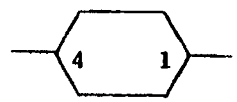 | 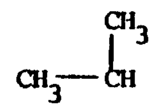 |
| 6 | 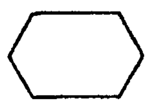 | 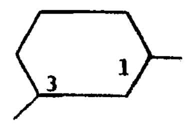 | 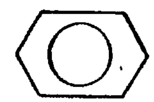 |

| Example No. | 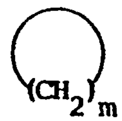 | 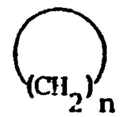 | R |
|---|---|---|---|
| 7 | 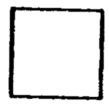 | 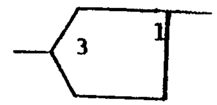 | $CH_2—CH=CH_2$ |
| 8 | 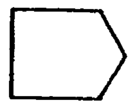 | 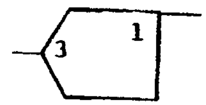 | 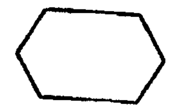 |
| 9 | 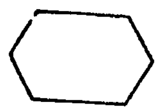 | 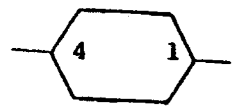 | 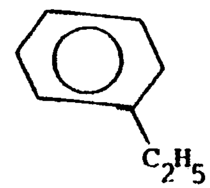 |
| 10 | 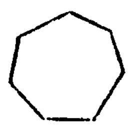 | 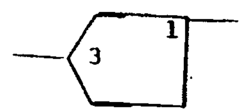 | $C_2H_5$ |

| Example No. | 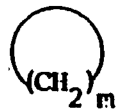 | 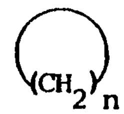 | R |
|---|---|---|---|
| 11 | 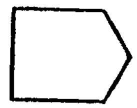 | 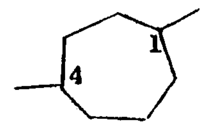 | $CH_3$ |
| 12 | 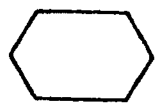 | 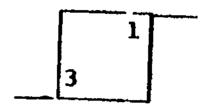 | $C_3H_7$ |
| 13 | 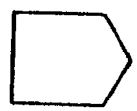 | 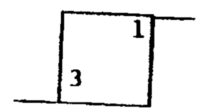 | $C_7H_{15}$ |
| 14 | 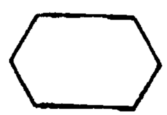 | 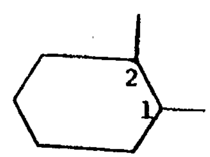 | $C_2H_5$ |

| Example No. | 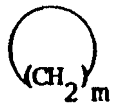 | 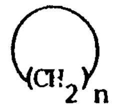 | R |
|---|---|---|---|
| 15 | 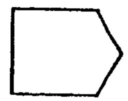 | 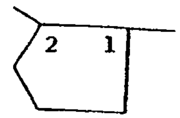 | $CH_3$ |
| 16 | 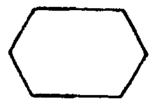 | 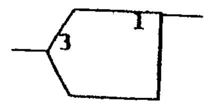 | 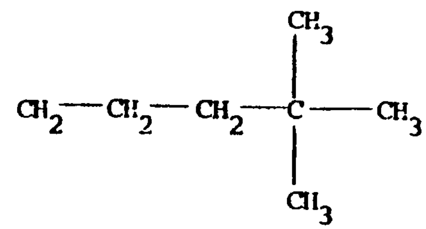 |
| 17 | 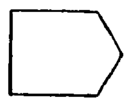 | 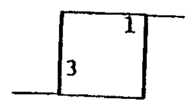 | $OCH_3$ |
| 18 | 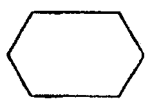 | 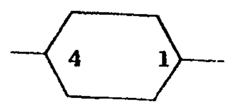 | $SCH_3$ |

| Example No. | 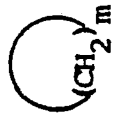 | 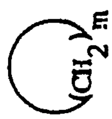 | R |
|---|---|---|---|
| 19 | 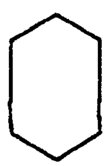 | 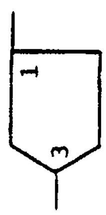 | $CH_2—CH_2—CH_2—CH=CH_2$ |
| 20 | 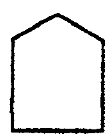 | 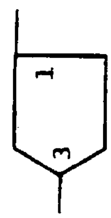 | H |

## Claims

**1.** A compound of the formula

I

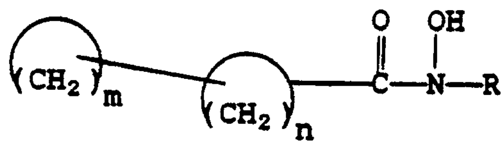

wherein

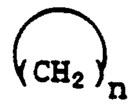 and 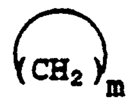

are cycloalkyl groups which may be the same or different, where m is an integer from 3 to 12 and n is an integer from 3 to 12; and

R is H, lower alkyl, aryl, lower alkenyl, cycloalkyl or aralkyl;

including pharmaceutically acceptable salts thereof.

2. A compound of claim 1 wherein

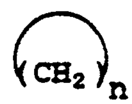

is substituted at the 2, 3 or 4 position with

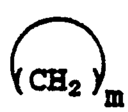.

3. A compound of claim 1 wherein m and n are integers from 3 to 8.

4. A compound of claim 1 wherein m and n are each 6.

5. A compound of claim 4 wherein R is lower alkyl.

6. A compound of claim 5 wherein R is methyl.

7. A compound of claim 5 wherein R is 1,1-dimethylethyl.

8. The compound of claim 1 having the name 4-cyclohexyl-N-hydroxy-N-methylcyclohexane-carboxamide.

9. The compound of claim 1 having the name 4-cyclohexyl-N-(1,1-dimethylethyl)-N-hydroxy-cyclohexanecarboxamide.

10. A composition for inhibiting allergic conditions in a mammalian species comprising an effective amount of a compound as defined in claim 1, or a pharmaceutically acceptable salt thereof, and a pharmaceutically acceptable carrier therefor.

11. Use of a compound as defined in claim 1 or of a pharmaceutically acceptable salt thereof, in preparing

a drug useful of inhibiting $\Delta^{5}$-lipoxygenase in a mammalian host.

**12.** Use of a compound as defined in claim 1 or of a pharmaceutically acceptable salt thereof, for preparing a drug for treating asthma in a mammalian species in need of such treatment.

## Revendications

**1.** Composé de formule

I

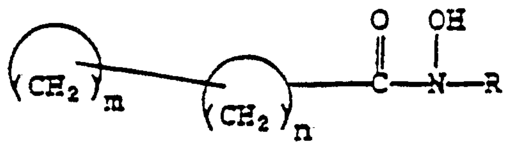

dans laquelle

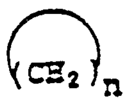 et 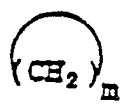

sont des radicaux cycloalkyle qui peuvent être identiques ou différents, m étant un nombre entier de 3 à 12 et n un nombre entier de 3 à 12 ; et

R est l'hydrogène ou un radical alkyle inférieur, aryle, alcényle inférieur, cycloalkyle ou aralkyle ;

y compris les sels pharmaceutiquement acceptables de ce composé.

**2.** Composé selon la revendication 1, dans lequel

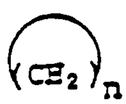

est substitué en position 2, 3 ou 4 par

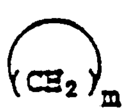.

**3.** Composé selon la revendication 1, dans lequel m et n sont des nombres entiers de 3 à 8.

**4.** Composé selon la revendication 1, dans lequel m et n sont tous deux égaux à 6.

**5.** Composé selon la revendication 4, dans lequel R est un radical alkyle inférieur.

**6.** Composé selon la revendication 5, dans lequel R est le radical méthyle.

**7.** Composé selon la revendication 5, dans lequel R est le radical 1,1-diméthyléthyle.

**8.** Composé selon la revendication 1, qui est le 4-cyclohexyl-N-hydroxy-N-méthylcyclohexanecarboxamide.

9. Composé selon la revendication 1, qui est le 4-cyclohexyl-N-(1,1-diméthyléthyl)-N-hydroxycyclohexanecarboxamide.

10. Composition permettant d'inhiber les états allergiques chez une espèce mammifère, comprenant une quantité efficace d'un composé tel qu'il est défini dans la revendication 1, ou d'un sel pharmaceutiquement acceptable de celui-ci, et un porteur pharmaceutiquement acceptable.

11. Utilisation d'un composé tel qu'il est défini dans la revendication 1, ou d'un sel pharmaceutiquement acceptable de celui-ci, pour préparer un médicament utilisable pour inhiber la $\Delta^5$-lipoxygénase chez un mammifère-hôte.

12. Utilisation d'un composé tel qu'il est défini dans la revendication 1, ou d'un sel pharmaceutiquement acceptable de celui-ci, pour préparer un médicament de traitement de l'asthme chez une espèce mammifère nécessitant un tel traitement.

## Patentansprüche

1. Verbindung der Formel I

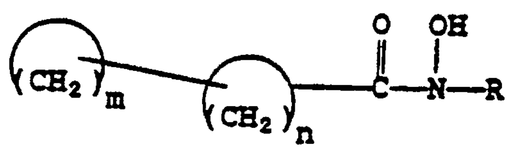

(I)

in der

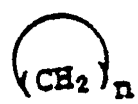

und

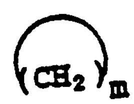

Cycloalkylreste darstellen, die gleich oder verschieden sein können, wobei m eine ganze Zahl von 3 bis 12 ist und n eine ganze Zahl von 3 bis 12 ist; und

R ein Wasserstoffatom, einen Niederalkyl-, Aryl-, Niederalkenyl-, Cycloalkyl- oder Aralkylrest darstellt; einschließlich der pharmazeutisch verträglichen Salze davon.

2. Verbindung nach Anspruch 1, in der

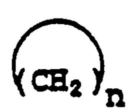

in 2-, 3- oder 4-Stellung mit

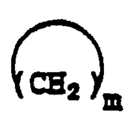

substituiert ist.

3. Verbindung nach Anspruch 1, in der m und n ganze Zahlen von 3 bis 8 sind.

4. Verbindung nach Anspruch 1, in der m und n jeweils 6 sind.

5. Verbindung nach Anspruch 4, in der R einen Niederalkylrest bedeutet.

6. Verbindung nach Anspruch 5, in der R eine Methylgruppe bedeutet.

7. Verbindung nach Anspruch 5, in der R eine 1,1-Dimethylethylgruppe bedeutet.

8. Verbindung nach Anspruch 1 mit der Bezeichnung 4-Cyclohexyl-N-hydroxy-N-methylcyclohexan-carboxamid.

9. Verbindung nach Anspruch 1 mit der Bezeichnung 4-Cyclohexyl-N-(1,1-dimethylethyl)-N-hydroxycyclohexancarboxamid.

10. Mittel zur Hemmung von allergischen Erkrankungen in einem Säuger, umfassend eine wirksame Menge einer Verbindung nach Anspruch 1 oder ein pharmazeutisch verträgliches Salz davon und einen pharmazeutisch verträglichen Träger dafür.

11. Verwendung einer Verbindung nach Anspruch 1 oder eines pharmazeutisch verträglichen Salzes davon zur Herstellung eines Arzneimittels, das zur Inhibierung der $\Delta^5$-Lipoxygenase in einem Säuger-Wirt geeignet ist.

12. Verwendung einer Verbindung nach Anspruch 1 oder eines pharmazeutisch verträglichen Salzes davon zur Herstellung eines Arzneimittels zur Behandlung von Asthma bei einem Säuger, der eine derartige Behandlung benötigt.